# EUROPÄISCHE PATENTSCHRIFT

(11) **EP 0 987 000 B1**
(45) Veröffentlichungstag und Bekanntmachung des Hinweises auf die Patenterteilung: **07.11.2001**
(21) Anmeldenummer: 99116324.7
(22) Anmeldetag: 19.08.1999
(51) Int. Cl.: A61K 7/00, A61K 7/50, A61K 7/06

(54) **Aerosol-Haarwaschmittel**
Aerosol shampoo
Shampoo sous forme d'aérosol

(30) Priorität: 10.09.1998 DE 19841339
(43) Veröffentlichungstag der Anmeldung: 22.03.2000
(73) Patentinhaber: GOLDWELL GmbH, 64297 Darmstadt (DE)
(72) Erfinder: Dubowoj, Polina, 64319 Pfungstadt (DE)

(56) Entgegenhaltungen:
- WO-A-92/07546
- WO-A-96/03969
- WO-A-97/20626

## Beschreibung

Die Erfindung betrifft ein Aerosol-Haarwaschmittel mit verbessertem Gebrauchseigenschaften.

Von einem guten Haarwaschmittel erwartet der Verbraucher vor allem eine zufriedenstellende Reinigungswirkung, eine volle cremige Schaumentwicklung bei der Anwendung, sparsamen Verbrauch, gute Haut- und Schleimhautverträglichkeit ohne jedwede hautreizende oder allergisierende Wirkung und gegebenenfalls auch einen haarkonditionierenden Effekt.

Es sind zahlreiche Shampoos bekannnt, die sich bemühen, diesen Anforderungen gerecht zu werden, ohne dies allerdings immer zu erreichen.

In der älteren deutschen Patentanmeldung Nr. 198 18 737.8 wird ein Shampoo zur Verfügung gestellt, das die obengenannten Vorraussetzungen erfüllt.

Dieses Haarwaschmittel enthält in wäßriger Grundlage 5 bis 50 Gew.-%, bezogen auf die Gesamtzusammensetzung, mindestens eines anionischen, amphoteren, zwitterionischen und/oder nichtionischen Tensids, sowie ein Treibmittelgemisch aus mindestens einem niedrigsiedenden Kohlenwasserstoff-Treibmittel und Kohlendioxid, und ist frei von Konservierungsmitteln sowie, gemäß einer bevorzugten Ausführungsform der Erfindung, auch frei von Parfumbestandteilen.

Die Erfindung hat sich die Aufgabe gestellt, dieses Shampoo noch zu verbessern. Dabei wurde gefunden, daß sich ein Aerosolshampoo mit einfacher Zusammensetzung und ausgezeichnetem Schaumvermögen sowie guten haarkonditionierenden Eigenschaften, das konservierungsmittelfrei ist, auch dann herstellen läßt, wenn anstelle des in der erwähnten älteren Anmeldung im dort beschriebenen Treibmittelgemisch zwingend vorhandenen Kohlendioxids ein Treibmittel benutzt wird, das ausschließlich aus niederen Kohlenwasserstoffen, wie Propan, Butan und/oder Isobutan besteht.

Gegenstand der Erfindung ist demgemäß ein Aerosol-Haarwaschmittel, das mindestens 60 Gew.-%, berechnet auf die Gesamtzusammensetzung, Wasser, nicht mehr als 20 Gew.-%, berechnet auf die Gesamtzusammensetzung, mindestens eines Tensids, mindestens eine haarkonditionierende Substanz und als Treibmittel mindestens einen niederen Kohlenwasserstoff enthält und frei von Konservierungsmitteln ist.

Aerosol-Shampoos sind im Prinzip bereits seit langem bekannt.

Nach den Ausführungen in der Monografie von K. Schrader, Grundlagen und Rezepturen der Kosmetika, 2. Aufl. (1989), S. 897, hat diese Produktgruppe jedoch "keine Daseinsberechtigung", da einmal die Verpackung relativ teuer sei, zum anderen die Entsorgung dieses Materials eine Umweltbelastung darstelle und vor allem noch große Probleme hinsichtlich möglicher Korrosionen bestünden.

Um so überraschender war es daher, daß die erfindungsgemäßen Zusammensetzungen weder korrodierend wirken noch sonst irgendwelche Nachteile aufweisen, sondern, ganz im Gegenteil, die eingangs erwähnten Vorteile besitzen und auch noch eine leichte Verteilung als Aerosolschaum über das gesamte zu waschende Haar und somit eine Optimierung der Reinigungswirkung erlauben.

Aus den WO 98/27 935, 98/27 936, 98/27 937 und 98/27 938 A1 sind bereits Aerosolshampoos bekannt, die vor allem eine verbesserte Ablagerung eines haut- und haarkonditionierenden Mittels aufdem Haar bzw. der Haut ermöglichen, sollen. Dazu sollen diese Mittel nicht mehr als 25, vorzugsweise bis zu 15 Gew.-% Wasser und mindestens 18 Gew.-% Tensid enthalten, was naturgemäß zu sehr konstenintensiven Produkten führt.

Es war daher völlig überraschend und für den Fachmann angesichts dieser Lehre nicht vorhersehbar, daß die erfindungsgemäßen Zusammensetzungen einen ausgezeichneten haarkonditionierenden Effekt ergeben.

Als niedrigsiedende Kohlenwasserstoff-Treibmittel werden insbesondere Propan, n-Butan und/oder Isobutan, vorzugsweise im Gemisch untereinander, eingesetzt. Als ergänzender Bestandteil können weitere niedrigsiedende Kohlenwasserstoffe, insbesondere n-Pentan, mitverwendet werden.

Das Gesamtverhältnis von Shampoo-Zusammensetzung zu Treibmittel liegt vorzugsweise bei etwa 10:1 bis 20:1, insbesondere bei etwa 12:1 bis etwa 16:1.

Die erfindungsgemäßen Zusammensetzungen enthalten, mit Ausnahme von Konservierungsmitteln und gegebenenfalls Parfumbestandteilen, die in Shampoos üblichen Stoffe.

Solche sind insbesondere Tenside, bevorzugt anionische Tenside.

Geeignete anionaktive Tenside im Rahmen der Erfindung sind in einer Menge von maximal 20, vorzugsweise mindestens 5 bis etwa 15 Gew.-%, der Zusammensetzung enthalten.

Dabei handelt es sich um solche vom Sulfat-, Sulfonat-, Carboxylat- und Alkylphosphat-Typ, vor allem natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettige Mono- und Dialkylphosphate, die milde, hautverträgliche Detergentien darstellen.

Im Rahmen der Erfindung weiterhin geeignete anionische Tenside sind α-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

R―(C₂H₄O)ₙ―O―CH₂COOX

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 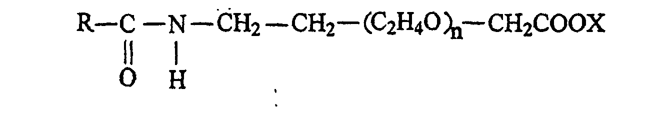 worin R und X die vorstehend angegebene Bedeutung haben, und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO® " und "AKYPO-SOFT® ".
Auch C₈-C₂₀-Acylisethionate können, allein oder im Gemisch mit anderen Tensiden, eingesetzt werden, ebenso Sulfofettsäuren und deren Ester.

Es können auch Mischungen aus mehreren anionischen Tensiden verwendet werden, beispielsweise ein Gemisch aus einem α-Olefinsulfonat und einem Sulfosuccinat, vorzugsweise im Verhältnis von 1:3 bis 3:1, oder einem Ethersulfat und einer Polyethercarbonsäure oder Alkylamidoethercarbonsäure.

Eine Übersicht über die in flüssigen Körperreinigungsmitteln zum Einsatz gelangenden anionaktiven Tenside findet sich im übrigen in der Monografie von K.Schrader, "Grundlagen und Rezepturen der Kosmetika", 2. Aufl. (1989, Hüthig Buchverlag), S. 595-600 und S. 683 bis 691.

Der besonders bevorzugte Mengenbereich an anionischen Tensiden in den erfindungsgemäßen flüssigen Shampoos liegt zwischen etwa 5 und etwa 15 Gew.-%, insbesondere bei etwa 7,5 bis etwa 12 Gew.-%, besonders bevorzugt bei etwa 10 Gew.-%, berechnet auf die Gesamtzusammensetzung des Mittels.

Weitere geeignete anionische Tenside sind auch C₈-C₂₂-Acylaminocarbonsäuren bzw. deren wasserlösliche Salze, vorzugsweise in einer Menge von 0,5 bis 5, insbesondere 1 bis 2,5 Gew.-%, berechnet auf die Gesamtzusammensetzung. Besonders bevorzugt sind N-Lauroylglutamat, insbesondere als Natriumsalz, sowie beispielsweise N-Lauroylsarcosinat, N-C₁₂-C₁₈-Acylasparaginsäure, N-Myristoylsarcosinat, N-Oleoylsarcosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze, vorzugsweise im Gemisch mit den obengenannten anionaktiven Tensiden.

Weitere geeignete Tenside in den erfindungsgemäßen Haarwaschmitteln sind nichtionische Tenside, vorzugsweise im Gemisch mit anionaktiven und/oder zwitterionischen bzw. amphoteren Tensiden.

Diese sind bei Schrader, l.c., auf den Seiten 600-601 und S. 694-695 beschrieben.

Geeignet sind insbesondere Alkylpolyglucoside mit der allgemeinen Formel

R―O―(R¹O)ₙ―Zₓ

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 5 bedeuten.

Diese Alkylpolyglucoside sind in letzter Zeit insbesondere als ausgezeichnete hautverträgliche schaumverbessernde Mittel in flüssigen Wasch- und Körperreinigungsmitteln bekannt geworden und sind in einer Menge von etwa 1 bis 10, insbesondere etwa 2,5 bis 5 Gew.-% der Gesamtzusammensetzung enthalten.

Gemische aus anionaktiven Tensiden und Alkylpolyglucosiden sowie deren Verwendung in flüssigen Körperreinigungsmitteln sind an sich bereits bekannt, beispielsweise aus der EP-A 70 074. Die dort beschriebenen Alkypolyglucoside sind prinzipiell auch im Rahmen der vorliegenden Erindung geeignet; ebenso die aus der EP-A 358 216 bekannten Gemische aus Sulfosuccinaten und Alkylpolyglucosiden.

Weitere nichtionische Tensidbestandteile sind beispielsweise langkettige Fettsäuremono- und -dialkanolamide, wie Cocosfettsäuremonoethanolamid und Myristinfettsäuremonoethanolamid, die auch als Schaumverstärker eingesetzt werden können.

Andere zusätzlich mitverwendbare nichtionische Tenside sind z.B. die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.

Weitere zusätzlich einsetzbare Tenside sind Aminoxide in einer Menge von etwa 0,25 bis etwa 5, vorzugsweise etwa 0,5 bis etwa 2,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder - ethylaminoxide, C₁₂-C₁₈-Alkyldi(hydroxyethyl)- oder -(hydroxypropyl)aminoxide, oder auch Aminoxide mit Ethylenoxid- und /oder Propylenoxidgruppen in der Alkylkette.

Solche Aminoxide sind beispielsweise unter den Bezeichnungen "Ammonyx® ", "Aromox® " oder "Genaminox® " im Handel.

Die erfindungsgemäßen Zusammensetzungen können als weiteren Tensid-Bestandteil amphotere bzw. zwitterionische Tenside, beispielsweise in einer Menge von etwa 0,25 bis etwa 7,5, vorzugsweise von etwa 1 bis etwa 5 Gew.-%, bezogen auf die Gesamtzusammensetzung, enthalten, ebenfalls vorzugsweise im Gemisch mit anionischen und/oder nichtionischen Tensiden.

Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.

### Im einzelnen können Betaine der Struktur

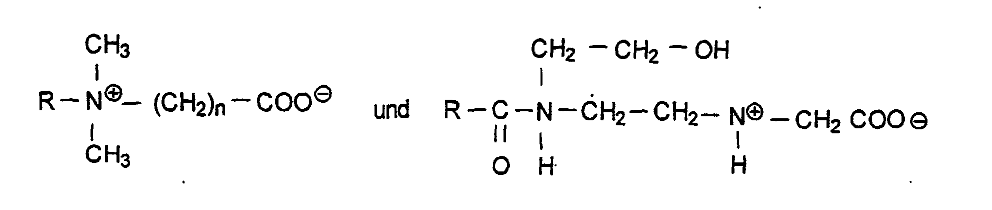

wobei R eine C₈-C₁₈-Alkylgruppe und n 1 bis 3 bedeuten,
Sulfobetaine der Struktur 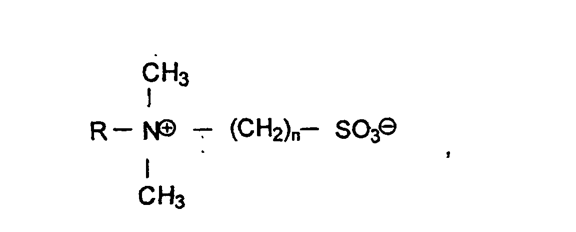 wobei R eine C₈-C₁₈-Alkylgruppe und n 1 bis 3 bedeuten,
und Amidoalkylbetaine der Struktur 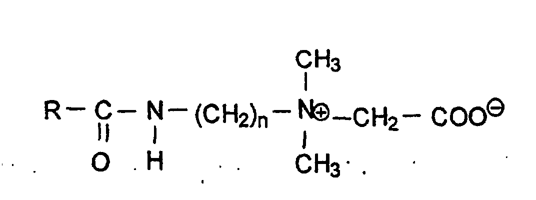 wobei R eine C₈-C₁₈-Alkylgruppe und n 1 bis 3 bedeuten, verwendet werden.

Die erfindungsgemäßen Shampoos enthalten auch konditionierende Wirkstoffe in einer Mengen zwischen etwa 0,05 und 2,5, vorzugsweise etwa 0,1 und 1,5 Gew.-% der Gesamtzusammensetzung. Solche sind beispielsweise Eiweißhydrolysate und Polypeptide, z.B., Keratinhydrolysate, Kollagenhydrolysate vom Typ "Nutrilan^{R}" oder Elastinhydrolysate sowie insbesondere auch pflanzliche, gegebenenfalls kationisierte Eiweißhydrolysate, z.B. "Gluadin^{R}", enthalten.

Bevorzugte konditionierende Zusätze sind haarkonditionierende Polymere, insbesondere kationische Polymere.

Dies sind z.B. die altbekannten quaternären Cellulosederivate des Typs "Polymer JR" sowie quaternisierte Homo- und Copolymere des Dimethyldiallylammoniumchlorids, wie sie unter dem Handelsnamen "Merquat® " im Handel sind, quaternäre Vinylpyrrolidon-Copolymere, insbesondere mit Dialkylaminoalkyl(meth)acrylaten, wie sie unter dem Namen "Gafquat® " bekannt sind, Copolymerisate aus Vinylpyrrolidon und Vi-nylimidazoliniummethochlorid, die unter dem Handelsnamen "Luviquat® " angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniumverbindungen der in der US-PS 4 157 388 beschriebenen Harnstoff-Struktur, die unter dem Handelsnamen "Mirapol® A 15" im Handel sind.

Verwiesen wird in diesem Zusammenhang auch auf die in den DE-OSen 25 21 960, 28 11010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerereingesetzt werden.

Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Anstelle oder zusätzlich zu den kationischen Polymeren können als haarkonditionierende PoIymere auch nichtionische und/oder anionische und/oder amphotere Polymere in den genannten Mengen eingesetzt werden.

Nichtionische Polymere sind beispielsweise alkohol- und/oder wasserlösliche Vinylpyrrolidon-Polymere wie ein Vinylpyrrolidon-Homopolymerisat oder -Copolymerisat, insbesondere mit Vinylacetat, sein.

Geeignete Vinylpyrrolidon-Polymere sind z.B. die unter dem Handelsnamen "Luviskol® " bekannten Produkte, beispielsweise die Homopolymerisate "Luviskol® K 30, K 60 und K 90" sowie die wasser- bzw. alkohollöslichen Copolymerisate aus Vinylpyrrolidon und Vinylacetat, die unter dem Handelsnamen "Luviskol® VA 55 bzw. VA 64" von der BASF AG vertrieben werden.

Weitere geeignete nichtionische Polymere sind Vinylpyrrolidon/Vinylacetat/Vinylpropionat-Copolymere wie "Luviskol® VAP 343", Vinylpyrrolidon/(Meth)Acrylsäureester-Copolymere sowie Chitosan-Derivate. Ihr Anteil in den erfindungsgemäßen Haarwaschmitteln liegt, wenn vorhanden, zwischen etwa 0,05 und etwa 5, vorzugsweise 0,1 und 2,5, insbesondere etwa 0,15 bis 1,5 Gew.-%, bezogen auf die Gesamtzusammensetzung des Mittels.

Als amphotere Polymere, die allein oder im Gemisch mit mindestens einem weiteren kationischen, nichtionischen oder anionischen Polymeren zum Einsatz gelangen, seien insbesondere Copolymerisate aus N-Octylacrylamid, (Meth)Acrylsäure und tert.-Butylaminoethylmethacrylat vom Typ "Amphomer® "; Copolymerisate aus Methacryloylethylbetain und Alkylmethacrylaten vom Typ "Yukaformer® ", z. B. das Butylmethacrylat-Copolymere "Yukaformer® Am75"; Copolymerisate aus Carboxylgruppen und Sulfongruppen enthaltenden Monomeren, z. B. (Meth)Acrylsäure und Itaconsäure, mit basische Gruppen, insbesondere Aminogruppen, enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der USA 3,927,199 bekannten Copolymeren genannt.

Geeignete anionische Polymere sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez® AN oder ES" vertrieben werden. Diese Polymeren können auch teilverestert sein, beispielsweise "Gantrez® ES 225", der Ethylester eines Ethylvinylethers/Maleinsäure-Copolymers, oder der Butyl- oder Isobutylester desselben.

Weitere geeignete anionische Polymere sind insbesondere Vinylacetat/Crotonsäure- oder Vinylacetat/Vinylneodecanoat/Crotonsäure-Copolymere des Typs "Resyn® "; Natriumacrylat/Vinylalkohol-Copolymere des Typs "Hydagen® F", Natriumpolystyrolsulfonat, z.B. "Flexan® 130"; Ethylacrylat/Acrylsäure/N-tert.-Butylacrylamid-Copolymere des Typs "Ultrahold® "; Vinylpyrrolidon/Vinylacetat/Itaconsäure-Copolymere, Acrylsäure/Acrylamid-Copolymere bzw. Natriumsalze derselben vom Typ "Reten® "; etc.

Schließlich können auch noch bekannte Polysiloxane als konditionierende Mittel in den erfindungsgemäßen flüssigen Haarwaschmitteln mitverwendet werden. Deren bevorzugter Anteil liegt dabei etwa zwischen 0,5 und etwa 5, insbesondere 1 bis 3 Gew.-% der Gesamtzusammensetzung. Geeignet sind sowohl leichtflüchtige als auch schwerflüchtige cyclische oder lineare Polysiloxane, beispielsweise die unter dem Trivialnamen "Dimethicone" bzw. "Phenyldimethicone" sowie "Cyclomethicone" bekannten Silikonöle.

Geeignet sind beispielsweise auch die in der EP-A 398 177 beschriebenen Silikonderivate, die dort in Kombination mit Alkylpolyglucosiden in flüssigen Detergens-Zusammensetzungen eingesetzt werden.

Weitere geeignete Konditioniermittel sind pflanzliche und tierische Öle sowie synthetische Fettsäureester, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven-bzw. Sojaöl, Lanolin und dessen Derivate, ebenso Mineralöle wie Paraffinöl und Vaseline.

Synthetische Öle und Wachse sind beispielsweise Silikonöle, Polyethylengykole, etc.

Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethlenglykol- und Polyglycerylfettsäureester wie PEG-7-glycerylcocoat, Cetylpalmitat, etc.

Eine Auflistung solcher Zusatzstoffe findet sich ebenfalls bei Schrader, l.c., auf S.695 bis 722.

Diese hydrophoben Komponenten sind in der erfindungsgemäßen Zusammensetzungen vorzugsweise in einer Gesamtmenge von etwa 0,5 bis etwa 7,5, insbesondere etwa 1 bis 5, berechnet auf die Gesamtzusammensetzung, enthalten.

Die erfindungsgemäßen Shampoos können selbstverständlich alle üblichen, in solchen Mitteln zum Einsatz gelangenden Stoffe enthalten.

Als solche seien beispielhaft Komplexbildner, Farbstoffe, pH-Regler, Viskositätsregler wie anorganische Salze, soweit sie nicht ohnehin in den Tensid-Ausgangsmischungen enthalten sind, Duftstoffe, Perlglanzmittel, Verdickungsmittel, Feuchthaltemittel, etc. genannt.

Ein weiterer bevorzugter Bestandteil ist Ethoxydiglykol, vorzugsweise in einer Menge von 0,1 bis 5 Gew.-% des erfindungsgemäßen Shampoos.

Gemäß einer bevorzugten Ausführungsform der Erfindung können die erfindungsgemäßen Shampoos auch Farbstoffe zur direkten oder oxidativen Färbung von Haaren enthalten, also sogenannte Tönungs- oder Färbeshampoos.

Besonders geeignet sind direktziehende Farbstoffe enthaltende Tönungsshampoos.

Es wurde nämlich festgestellt, daß solche Shampoos, die die erfindungsgemäße Tensidzusammensetzung und insbesondere kationische direktziehende Farbstoffenthalten, eine besonders brillante, glänzende und stabile Haarfärbung gegenüber bekannten Tönungsshampoos auf Basis konventioneller Tenside bzw. Tensidmischungen ergeben.

Der pH-Wert der erfindungsgemäßen Shampoos liegt im üblichen Bereich zwischen etwa 5 und 8,5; für Spezialprodukte kann er auch unterhalb 5 eingestellt werden.

Die Viskosität liegt im Bereich zwischen etwa 500 und etwa 1500 mPa•s bei 20 °C, vorzugsweise etwa 100 bis etwa 600, insbesondere 250 bis 500 mPa•s bei 20 °C, gemessen nach Brookfield oder Höppler bei einer Scherspannung von 10 s⁻¹.

Als Behälter zur Abgabe der erfindungsgemäßen Aerosol-Shampoos können sowohl Aluminiummonoblockdosen als auch Weißblechdosen, vorzugsweise mit Innenbeschichtung, z.B. auf Epoxyharz-Basis, versehen, als auch Kunststoffdosen verwendet werden.

Die Herstellung der erfindungsgemäßen Produkte erfolgt durch Zusammenrühren der einzelnen Komponenten in Wasser, wobei auch Vormischungen verschiedener Bestandteile verwendet werden können, und anschließendes Aufpressen des Treibmittels.

Das folgende Beispiel dient der Illustration der Erfindung.

100 Gewichtsteile einer wäßrigen Zusammensetzung aus

| | |
|---|---|
| 10,00 Gew.-% | Natriumlaurylethersulfat (∼2,5 EO) |
| 1,50 | Cocoamphodiacetat, Natriumsalz |
| 2,00 | C₁₂-C₁₄-Alkylpolyglucosid (P.D. ∼1,5) |
| 0,30 | Polymer JR 400 (Kationisches Cellulose-Polymer) |
| 0,70 | PEG-7-Glycerylcocoat |
| 0,05 | NaOH, 32 % |
| 0,45 | Citronensäuremonohydrat |
| 0,30 | Parfum |
| ad 100,00 | Wasser |

wurden mit 5 g eines handelsüblichen Propan/Butan-Treibmittelgemisches in eine Aerosoldose aus Aluminium abgefüllt.

Diese Shampoo-Zusammensetzung zeigte ein ausgezeichnetes Schaumvermögen und ließ sich leicht auf dem Haar verteilen. Bei Langzeitanwendung traten selbst bei Konservierungsmittel-Allergikern keine allergischen oder hautsensibilisierenden Effekte auf.

Es ist auch möglich, durch zusätzliches Weglassen der Parfumkomponente auch ein absolut reizfreies Shampoo für Parfumallergiker herzustellen.
Die Untersuchung der geöffneten Dosen mit dem Produkt nach dem obigen Beispiel zeigte nach 3 Monaten keinerlei Korrosionserscheinungen.
Darüberhinaus war, trotz der Abwesenheit von Konservierungsmitteln, keinerlei Keimbildung feststellbar.

Überrraschenderweise ergab sich auch eine eindeutige Präferenz der Zusammensetzung nach Beispiel 1 gegenüber einer gleichen Zusammensetzung, die nicht als Aerosolshampoo konfektioniert war, hinsichtlich des Glanzes, der Kämmbarkeit, der Glätte, der Lockerkeit, des Volumens und der Glätte des Haares im Doppelblindversuch an jeweils 10 Personen.

## Patentansprüche

1. Aerososol-Haarwaschmittel, enthaltend, berechnet auf die Gesamtzusammensetzung, mindestens 60 Gew.-% Wasser, maximal 20 Gew.-% mindestens eines anionischen, amphoteren, zwitterionischen und/oder nichtionischen Tensids, sowie mindestens eine haarkonditionierende Substanz, **dadurch gekennzeichnet, daß** es als ausschließliches Treibmittel mindestens einen niedrigsiedenden Kohlenwasserstoff enthält und frei von Konservierungsmitteln ist.

2. Aerosol-Haarwaschmittel nach Anspruch 1, **dadurch gekennzeichnet, daß** es als haarkonditionierende Substanz mindestens ein kationisches Polymer enthält.

3. Aerosol-Haarwaschmittel nach Anspruch 1 oder 2, **dadurch gekennzeichnet, daß** es maximal 15 Gew.-%, berechnet auf die Gesamtzusammensetzung, mindestens eines Tensids enthält.

## Claims

1. Aerosol shampoo composition, comprising, calculated to the total composition, at least 60 % by weight of water, at maximum 20% by weight of at least one anionic, amphoteric, zwitterionic and/or nonionic surfactant, as well as at least one hair-conditioning substance, comprising as exclusive propellant at least one low-boiling hydrocarbon, and being free from preservatives.

2. Aerosol shampoo composition according to claim 1, comprising as hair-conditioning substance at least one cationic polymer.

3. Aerosol shampoo composition according to claim 1 or 2, comprising a maximum of 15% by weight, calculated to the total composition, of at least one surfactant.

## Revendications

1. Shampooing en aérosol, contenant, rapporté à la composition totale, au moins 60 % en poids d'eau, au plus 20 % en poids d'au moins un agent tensioactif anionique, amphotère, zwiterrionique et/ou non ionique, ainsi qu'au moins un agent de conditionnement capillaire, **caractérisé en ce qu'**il contient comme agent propulseur exclusif, au moins un hydrocarbure à bas point d'ébullition et **en ce qu'**il ne contient aucun agent conservateur.

2. Shampooing en aérosol selon la revendication 1, **caractérisé en ce qu'**il contient au moins un polymère cationique en tant qu'agent de conditionnement capillaire.

3. Shampooing en aérosol selon la revendication 1 ou 2, **caractérisé en ce qu'**il contient, rapporté à la composition totale, au plus 15 % en poids d'au moins un agent tensioactif.
